# EUROPEAN PATENT SPECIFICATION

(11) **EP 3 958 860 B1**
(45) Date of publication and mention of the grant of the patent: **15.11.2023**
(21) Application number: 20850331.8
(22) Date of filing: 24.03.2020
(51) Int. Cl.: A61K 31/175, A61K 31/426, A61P 31/06, C07D 277/50, C07C 337/08

(54) **COMPOUNDS EXHIBITING ANTI-TUBERCULOSIS ACTIVITY**
VERBINDUNGEN MIT ANTI-TUBERKULOSE-AKTIVITÄT
COMPOSÉS PRÉSENTANT UNE ACTIVITÉ ANTI-TUBERCULOSE

(30) Priority: 02.08.2019 TR 201911755
(43) Date of publication of application: 02.03.2022
(73) Proprietor: Anadolu Universitesi, Tepebasi/Eskisehir (TR)
(72) Inventor: TURAN, Gülhan, Eskisehir (TR); DEMIREL, Müzeyyen, Eskisehir (TR); NAMLI, Irem, Eskisehir (TR)
(74) Representative: Sevinç, Cenk
(86) International application number: PCT/TR2020/050232
(87) International publication number: WO 2021/025644

(56) References cited:
- DATABASE HCAPLUS [Online] 1 December 1997 (1997-12-01), Turan-Zinouti Gulhan ET AL: "Some new 4-aryl-2-thiazolylhydrazones", XP055916326, retrieved from STN Database accession no. 1998:122846 & DATABASE REGISTRY [Online] CHEMICAL ABSTRACTS SERVICE, COLUMBUS, OHIO, US; 2 April 1998 (1998-04-02), XP55916330,
- TURAN-ZITOUNI G ET AL: "Synthesis and antituberculosis activity of new thiazolylhydrazone derivatives", EUROPEAN JOURNAL OF MEDICINAL CHEMISTRY, ELSEVIER, AMSTERDAM, NL, vol. 43, no. 5, 1 May 2008 (2008-05-01), pages 981-985, XP022639691, ISSN: 0223-5234, DOI: 10.1016/J.EJMECH.2007.07.001 [retrieved on 2008-05-01]
- MAILLARD, L. T. et al.: "Synthesis and anti-Candida activity of novel 2-hydrazino-1, 3-thiazole derivatives", Bioorganic & medicinal chemistry letters, vol. 23, no. 6, 2013, pages 1803-1807, XP028986052,
- SINGH, S. et al.: "Substituted hydrazinecarbothioamide as potent antitubercular agents: synthesis and quantitative structure-activity relationship (QSAR", Bioorganic & Medicinal Chemistry Letters, vol. 20, no. 8, 2010, pages 2597-2600, XP022639691,
- TURAN-ZITOUNI, G. et al.: "Synthesis and antituberculosis activity of some N-pyridyl-N'-thiazolylhydrazine derivatives", European journal of medicinal chemistry, vol. 45, no. 5, 2010, pages 2085-2088, XP026976534,
- OZADALI, K. et al.: "Synthesis and antimycobacterial activities of some new thiazolylhydrazone derivatives", Bioorganic & medicinal chemistry letters, vol. 24, no. 7, 2014, pages 1695-1697, XP028835181, DOI: 10.1016/j.bmcl.2014.02.052

## Description

### Technical Field

The invention relates to the compounds or pharmaceutically acceptable salts thereof, that exhibit anti-tuberculosis activity.

### Prior Art

According to the data of the World Health Organization, 9,2 million people suffer from tuberculosis annually and tuberculosis constitutes 2,5% of all of the diseases around the world and 26% of preventable deaths. Tuberculosis is usually caused by *Mycobacterium tuberculosis* (MTB). The bacteria *Mycobacterium avium complex (MAC) (Mycobacterium avium* and *Mycobacterium intracellulare)* results in the development of atypical tuberculosis infection. In particular, in the case of AIDS or cancer diseases, where the immune system weakens, the geriatric population and patients suffering from COPD (Chronic Obstructive Pulmonary Disease) may have a higher risk of death.

Medications that are used in tuberculosis treatment are mainly divided into two groups. The first group consists of; isoniazid (INH), rifampicin (RFM), pyrazinamide (PZA), ethambutol (ETM) and streptomycin (SM). The second group includes; more toxic and less tolerable medications such as cycloserine, ethionamide, kanamycin, capreomycin, and para-aminosalicylic acid. One of the most unfavorable cases in controlling tuberculosis is the development of drug resistance due to incorrect treatment or any other reasons. As MAC micro bacteria are naturally resistant to both of the groups, the development of novel compounds is seriously required.

The two compounds subject to the present invention and synthesis methods of these compounds have been disclosed in the articles "Some new 4-aryl-2-thiazolylhydrazones Turan-Zitouni, Gulhan; Kaplancikli, Z. Asim; Chevallet, PierreFarmaco (1997), 52(10), 635-638" and "Synthesis and anti-Candida activity of novel 2-hydrazino-1,3-thiazole derivatives Maillard, Ludovic T.; Bertout, Sebastien; Quinonero, Ophelie; Akalin, Gulsen; Turan-Zitouni, Gulhan; Fulcrand, Pierre; Demirci, Fatih; Martinez, Jean; Masurier, Nicolas Bioorganic & Medicinal Chemistry Letters (2013), 23(6), 1803-1807"

However, the prior art fails to clarify the anti-tuberculosis activities of these compounds and how it is ensured that Compound 2, which is water-insoluble can be dissolved in water.

Turan-Zinouti Gulhan et al. discloses some new 4-aryl-2-thiazolylhydrazones which are used as herbicides.

Turan-Zitounig et al. also synthesized new thiazolylhydrazone derivatives and showed antituberculosis activity of them.

Maillard, L. T. et al. synthesized novel 2- hydrazino-1, 3-thiazole derivatives and showed their anti-Candida activity.

### Brief Description and Aims of the Invention

It has been surprisingly observed in an *in vitro* anti-microbial activity assessment carried out for two compounds with the chemical structures thereof presented here below that these two compounds were at least five times more active on *Mycobacterium tuberculosis* H37Rv than Rifampicin. Compound 2 exhibited activity on *Mycobacterium avium* as well.

After the determination of activity, Compound 1, which is soluble in water, was subjected to *in vivo* animal assays and it has determined that this compound didn't exhibit toxicity. Compound 2, which is practically water-insoluble, was not subjected to *in vivo* tests.

Solubility ratios of Compound 2 in various organic solvents were determined and it has been observed in solubility assays carried out with solubility enhancing agents that this compound was dissolved in water to an extent with the use of hydroxypropyl-beta-cyclodextrin (HPβCD) or Polysorbate 80 (Tween 80).

### Figures

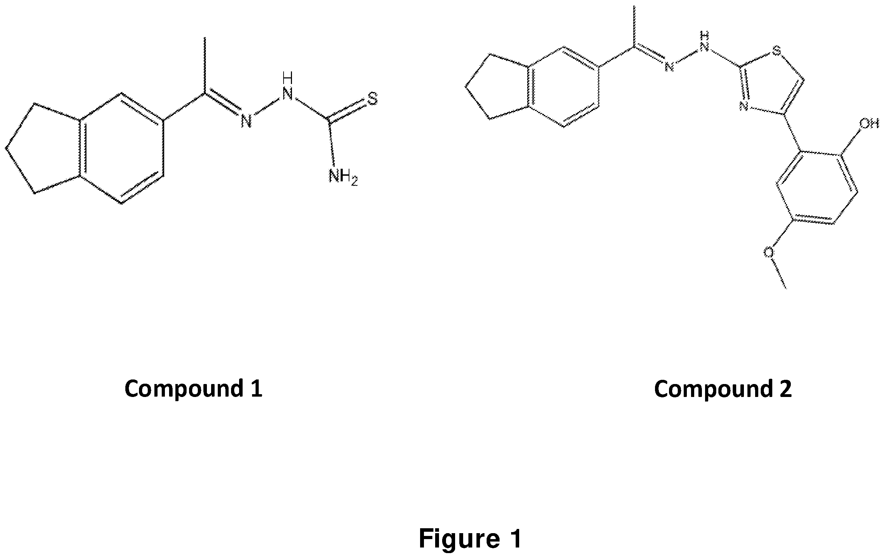

**Figure 1****:** Shows the chemical structures of Compound 1:2-[1-(2,3-dihydro-1H-inden-5-yl)ethylidene]hydrazinecarbothioamide and Compound 2:2-[2-(2-(1-(2,3-dihydro-1H-inden-5-yl)ethylidene)hydrazinyl)thiazol-4-yl]-4-methoxy phenol.

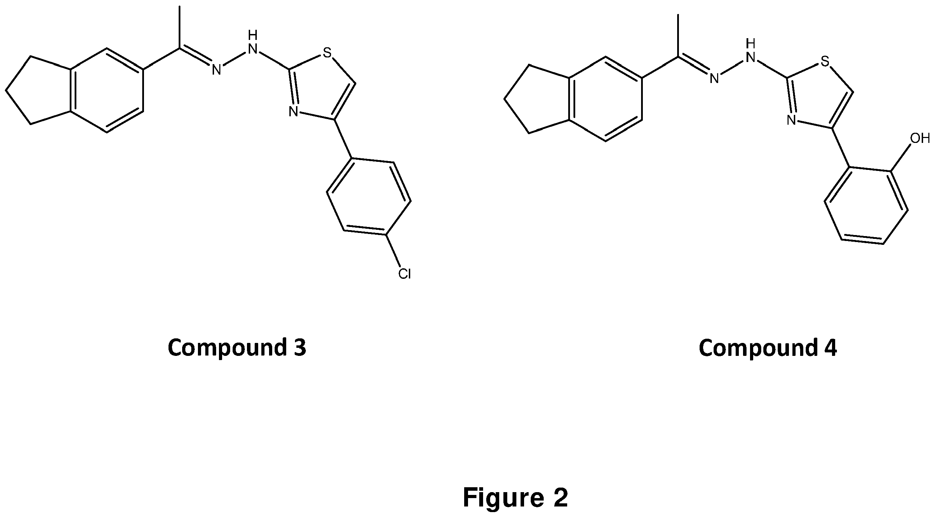

**Figure 2****:** Shows the chemical structures of Compound 3: 4-(4-Chlorophenyl)-2-[2-(1-(2,3-dihydro-1H-inden-5yl)ethylidene)hydrazinyl]thiazole and Compound 4: 2-[2-(2-(1-(2,3-Dihydro-1H-inden-5-yl)ethylidene)hydrazinyl)thiazol-4-yl]phenol

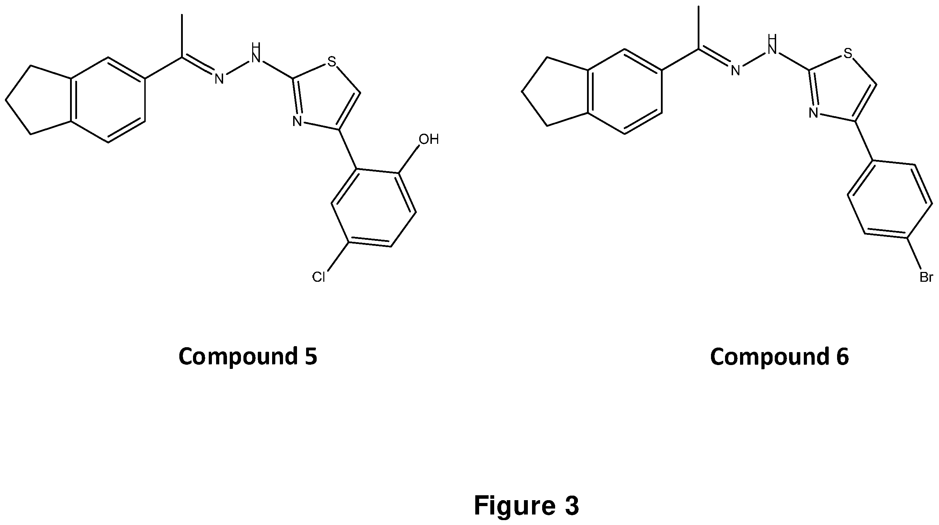

**Figure 3****:** Shows the chemical structures of Compound 5: 4-Chloro-2-[2-(2-(1-(2,3-dihydro-1H-inden-5 yl)ethylidene)hydrazinyl)thiazol-4-yl]phenol and Compound 6: 4-(4-Bromophenyl)-2-[2-(1-(2,3-dihydro-1H-inden-5-yl)ethylidene)hydrazinyl]thiazole

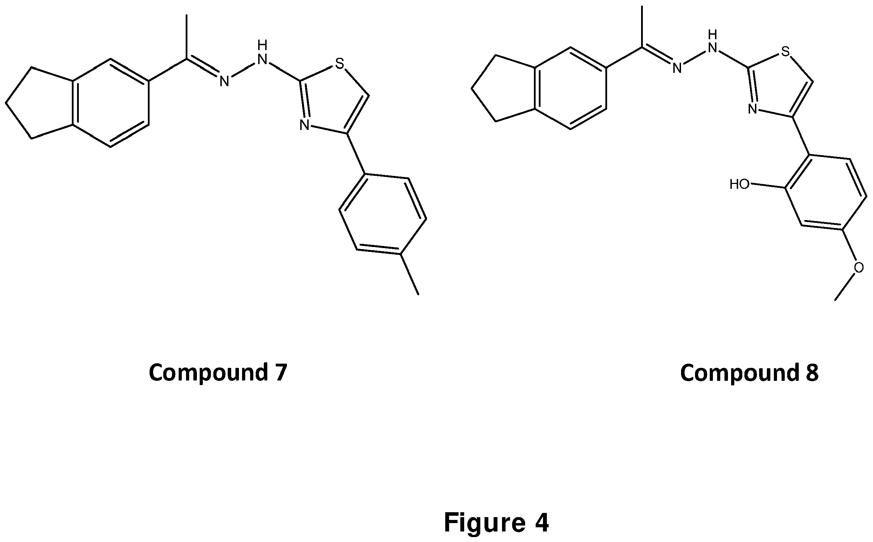

**Figure 4****:** Shows the chemical structures of Compound 7: 2-[2-(1-(2,3-Dihydro-1H-inden-5-yl)ethylidene)hydrazinyl)-4-(p-tolyl)]thiazole and Compound 8: 2-[2-(2-(1-(2,3-Dihydro-1H-inden-5-yl)ethylidene)hydrazinyl)thiazol-4-yl]-5-methoxyphenol

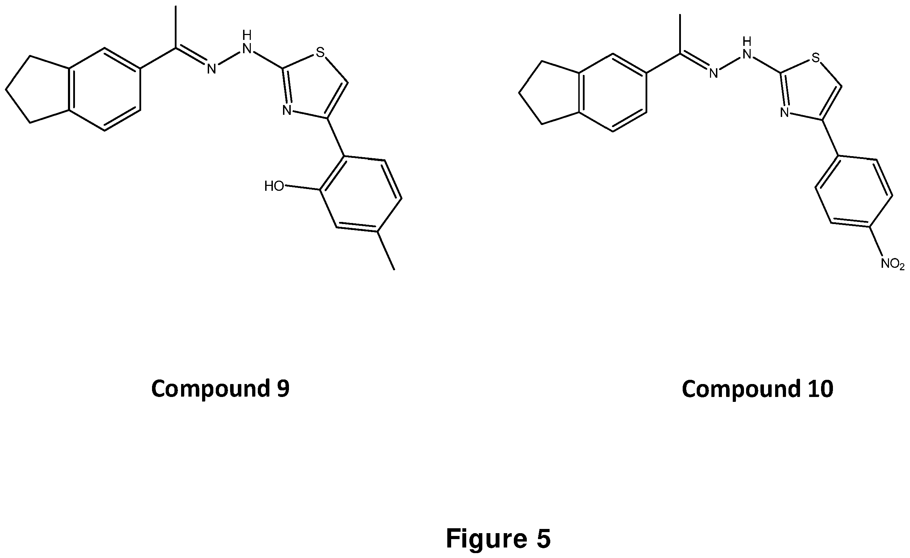

**Figure 5****:** Shows the chemical structures of Compound 9: 2-[2-(2-(1-(2,3-Dihydro-1H-inden-5-yl)ethylidene)hydrazinyl)thiazol-4-yl]-5-methylphenol and Compound 10: 2-[2-(1-(2,3-Dihydro-1H-inden-5-yl)ethylidene)hydrazinyl)-4-(4-nitrophenyl)]thiazole

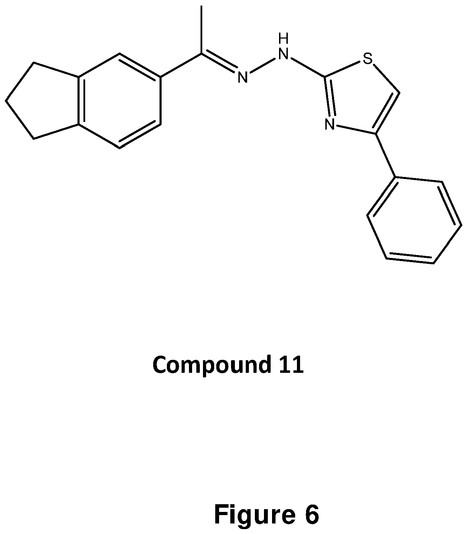

**Figure 6****:** Shows the chemical structures of Compound 11: 2-[2-(1-(2,3-Dihydro-1H-inden-5-yl)ethylidene)hydrazinyl]-4-phenylthiazole

### Detailed Description of the Invention

Chemical structures and chemical names of said compounds are given below.
**Compound 1:** 2-[1-(2,3-dihydro-1H-inden-5-yl)ethylidene]hydrazinecarbothioamide
**Compound 2:** 2-[2-(2-(1-(2,3-dihydro-1H-inden-5-yl)ethylidene)hydrazinyl)thiazole-4-yl]-4-methoxy phenol
**Compound 3:** 4-(4-Chlorophenyl)-2-[2-(1-(2,3-dihydro-1H-inden-5yl)ethylidene)hydrazinyl] thiazole
**Compound 4:** 2-[2-(2-(1-(2,3-Dihydro-1H-inden-5-yl)ethylidene)hydrazinyl)thiazol-4-yl]phenol
**Compound 5:** 4-Chloro-2-[2-(2-(1-(2,3-dihydro-1H-inden-5-yl)ethylidene)hydrazinyl)thiazol-4-yl]phenol
**Compound 6:** 4-(4-Bromophenyl)-2-[2-(1-(2,3-dihydro-1H-inden-5-yl)ethylidene)hydrazinyl] thiazole
**Compound 7: :** 2-[2-(1-(2,3-Dihydro-1H-inden-5-yl)ethylidene)hydrazinyl)-4-(p-tolyl)]thiazole
**Compound 8:** 2-[2-(2-(1-(2,3-Dihydro-1H-inden-5-yl)ethylidene)hydrazinyl)thiazol-4-yl]-5-**methoxyphenol**
**Compound 9:** 2-[2-(2-(1-(2,3-Dihydro-1H-inden-5-yl)ethylidene)hydrazinyl)thiazol-4-yl]-5-methylphenol
**Compound 10:** 2-[2-(1-(2,3-Dihydro-1H-inden-5-yl)ethylidene)hydrazinyl)-4-(4-nitrophenyl)] thiazole
**Compound 11:** 2-[2-(1-(2,3-Dihydro-1H-inden-5-yl)ethylidene)hydrazinyl]-4-phenylthiazole

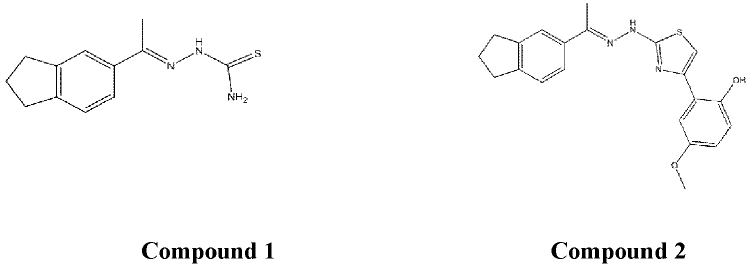

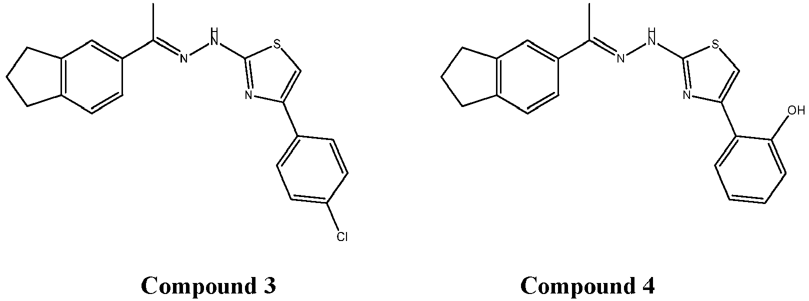

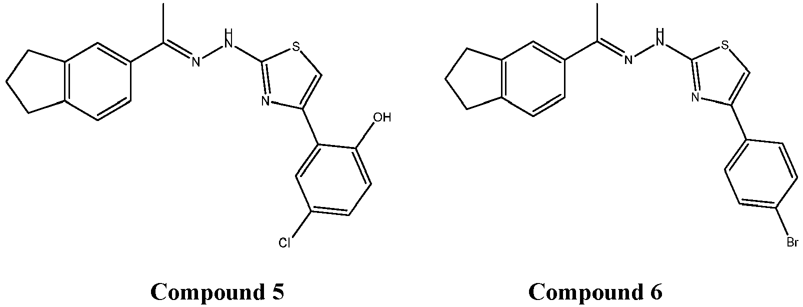

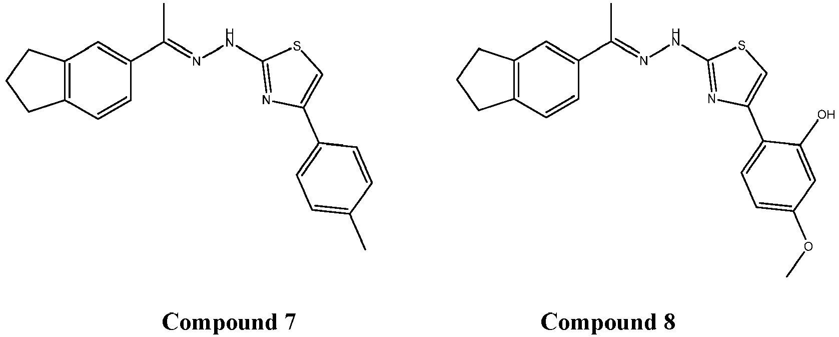

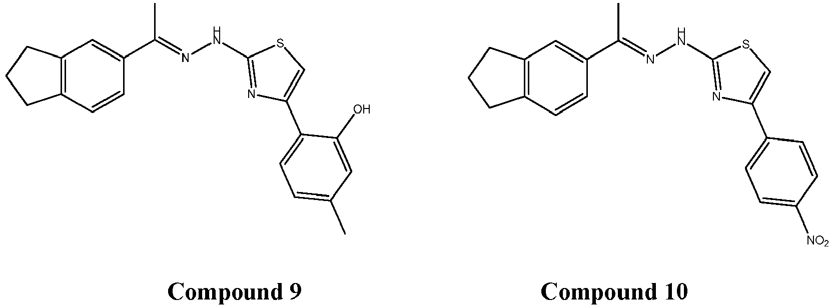

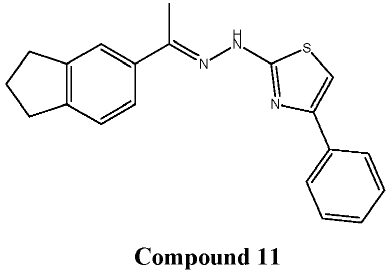

When both of the compounds were tested on *Mycobacterium tuberculosis* H37Rv (ATCC27294) with the BACTEC 460 Radiometric system used in the primary screening carried out for in vitro assessment of antimicrobial activity of Compounds in the TAACF research center (Tuberculosis Antimicrobial Acquisition & Coordinating Facility), Alabama/USA, they exhibited an inhibition effect higher than 90% ( Compound 1 99%, Compound 2 95%). Following this, to determine the actual minimum inhibitory concentration in the second phase, both of the compounds were retested to the lowest concentration of *Mycobacterium tuberculosis* H37Rv with the technique of MABA (Microplate Alamar Blue Assay) and MIC value for the 1^{st} compound was determined <0.03 µg/ml while this value was determined <0.05 µg/ml for the second compound. The result of Rifampicin subjected to the assay under the same conditions was determined as 0.25 µg/ml. The results obtained show that both of the Compounds are more active than Rifampicin.

### Compound 2 also exhibited the MIC concentration of 0.39 µg/ml on Mycobacterium avium with BACTEC.

Any conditions concerning toxicity have not been observed in 1000 mg/kg determined as the maximum tolerable dose in *in vivo* tests carried out for Compound 1.

Anti-tuberculosis activities of Compounds subject to the invention have been disclosed. The results of the activity obtained and presented below show that both of the Compounds were determined to be very active.

Supersaturated solutions of Compound 2 in various organic solvents were left to rest in an ultrasonic bath for 15 minutes at room temperature and thus solubility ratios thereof were quantitatively determined by the use of an analytical method and the results obtained were presented below Table 1.

**Table 1**

| Solvent | Solubility ratio | Solubility state |
|---|---|---|
| Dimethyl sulfoxide | more than 90 mg/ml | Very soluble |
| Methanol | 4.29 mg/ml | Poorly soluble |
| Ethanol | 3.17 mg/ml | Poorly soluble |
| Acetone | 1.02 mg/ml | Poorly soluble |
| Acetonitrile | 0.25 mg/ml | Very Poorly soluble |

To ensure that Compound 2 is dissolved in water, in the assays carried out with solubility enhancing cyclodextrin molecules, different cyclodextrins [hydroxypropyl beta-cyclodextrin (HP-β-CD), sulfobutyl ether beta-cyclodextrin (SBE-β-CD)], different methods [kneading, evaporation, lyophilization], different solvents (dimethyl sulfoxide, methanol, ethanol) were studied at the molar ratio of 1: 1 compound: cyclodextrin. In these assays, only in the evaporation method with HP-β-CD within ethanol, it has been determined that Compound 2 was dissolved in water at the ratio of 0.59 µg/ml and any significant increase in solubility was not observed.

In the assays carried out with the solubility enhancing Polysorbate 80 (Tween 80) in order to ensure water solubility of Compound 2, firstly solubility of the compound was determined in an aqueous solution prepared with a mass ratio of 1:6 (compound: Tween 80) and then the same mass ratio was studied with different solvents (ethanol, methanol) and different drying methods (evaporation, evaporation+lyophilization). In light of the results of solubility obtained and presented below Table 2, it has determined that the solution of the compound that was prepared with the Tween 80 in methanol medium had the highest solubility rate after evaporation.

**Table 2**

| Solvent | Method | The ratio of water solubility |
|---|---|---|
| Ethanol | Evaporation | 58,80 µg/ml |
| Methanol | Evaporation | 70,05 µg/ml |
| Methanol | Evaporation + Lyophilization | 46,23 µg/ml |
| Solubility ratio in the aqueous solution prepared with the mass ratio of 1:6 (compound: Tween 80) 42,44 µg/ml | | |

Details of the method where high solubility was determined with Polysorbate 80 (Tween 80) are presented below:
After the substances at the mass ratio of 1:6 (compound: Tween 80) were dissolved in 7 ml methanol, they were evaporated in a rotating evaporator at 175 RPM, at a temperature of 40°C and a vacuum of 140 atm. The thin film obtained was firstly blended with vortex adding 3 ml water, and then was left to rest for 15 minutes in an ultrasonic bath. The mixture obtained was filtered out with a plastic filter having a pore diameter of 0.2 µm and afterward solubility ratio of the mixture was determined with HPLC.

It has been determined with the following results that the compounds were at least five times more active than Rifampicin that is an antibiotic used in tuberculosis treatment (Table 3).

**Table 3**

| | Primary screening % inhibition | MIC H37Rv (µg/ml) | MIC *M. avium* (µg/ml) |
|---|---|---|---|
| Compound 1 | 99 | <0.03 | |
| Compound 2 | 95 | <0.05 | 0.39 |
| Compound 3 | 95 | <12.5 (0.39) | 0.78 |
| Compound 4 | 95 | <12.5 (0.20) | 0.78 |
| Compound 5 | 95 | <12.5 | |
| Compound 6 | 94 | <12.5 (0.39) | 1.56 |
| Compound 7 | 94 | <12.5 (0.20) | 0.2 |
| Compound 8 | 94 | <12.5 | |
| Compound 9 | 94 | <12.5 (0.05) | 0.1 |
| Compound 10 | 93 | <12.5 (1.56) | 3.13 |
| Compound 11 | 92 | <12.5 (3.13) | 12.5 |
| Rifampicin | 98 | =0.25 | |

These compounds provide an important advantage as they can be used in lower doses than the drugs used in current treatments, as these compounds exhibit very high activity.

## Claims

1. A compound for use in the treatment of tuberculosis, wherein the compound is selected from the group consisting of:

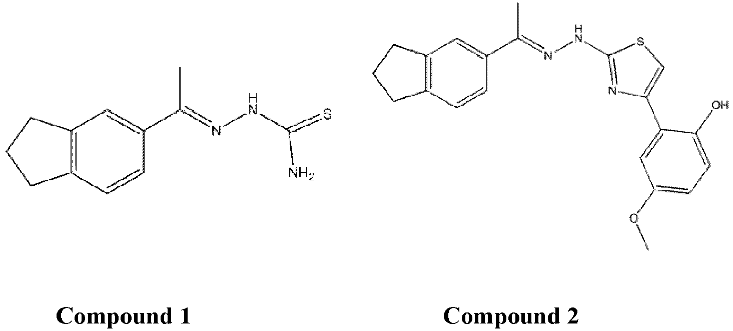

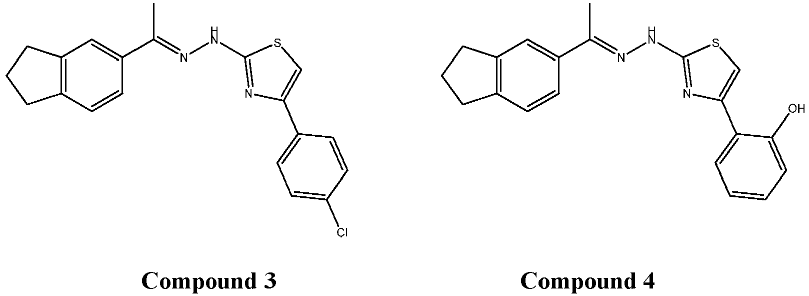

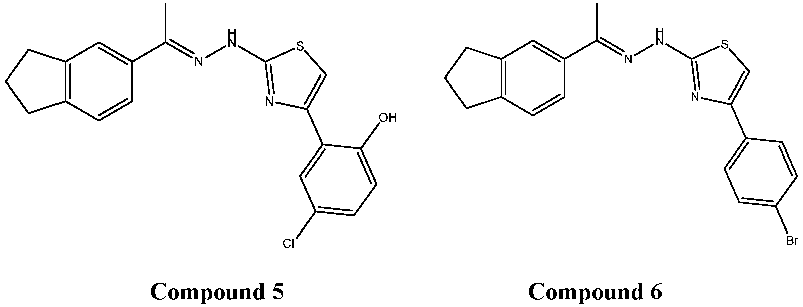

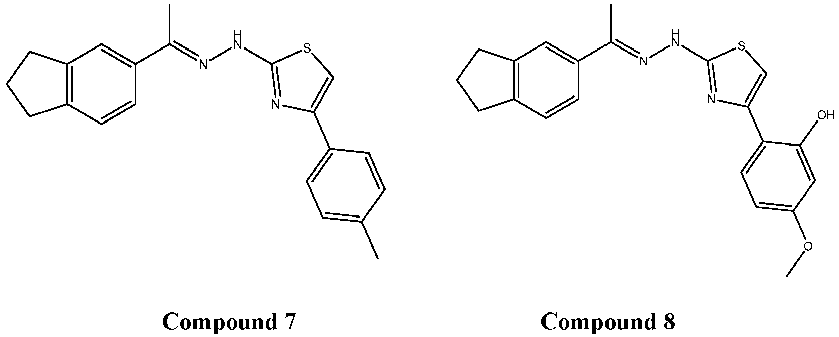

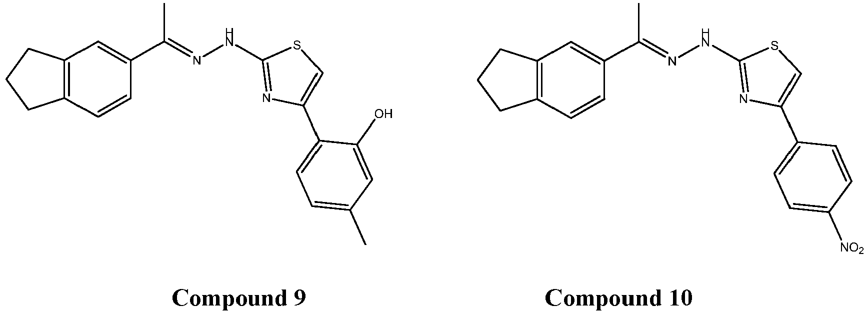

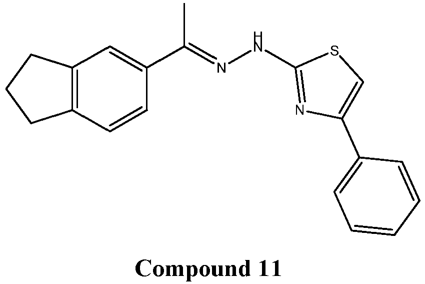

2. A compound for use according to claim 1 in antituberculosis treatment.

3. The method for dissolving compound of below structure in water, 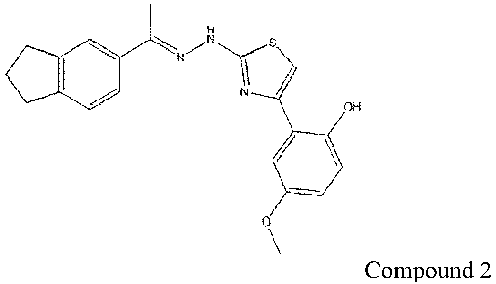 **Characterized by** comprising the process steps of;
• dissolving the substances of Compound 2: Polysorbate 80 (Tween 80) at the mass ratio of 1:6 in 7 ml methanol,
• evaporating them at 175 RPM, at a temperature of 40°C and in a vacuum of 140 ATM,
• adding water to the film obtained and mixing the film with vortex,
• leaving the film to rest for 15 minutes in an ultrasonic bath.

4. A compound for use according to claim 1; wherein the maximum dose of Compound 1 for anti-tuberculosis treatment is 1000 mg/kg.

## Patentansprüche

1. Eine Verbindung zur Verwendung bei der Behandlung von Tuberkulose, wobei die Verbindung ausgewählt ist aus der Gruppe bestehend aus:

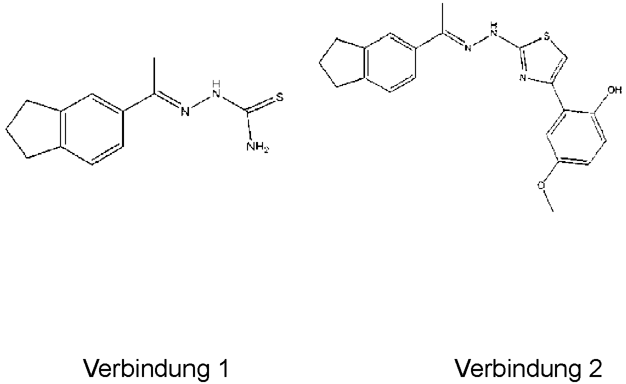

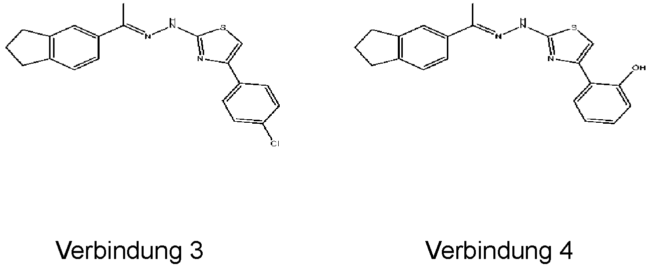

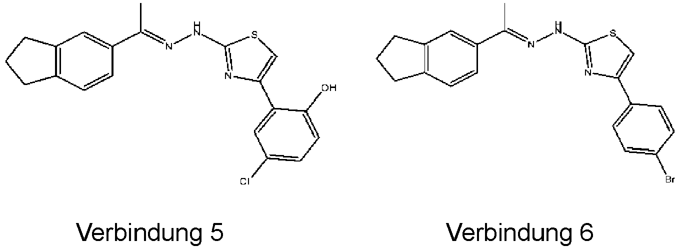

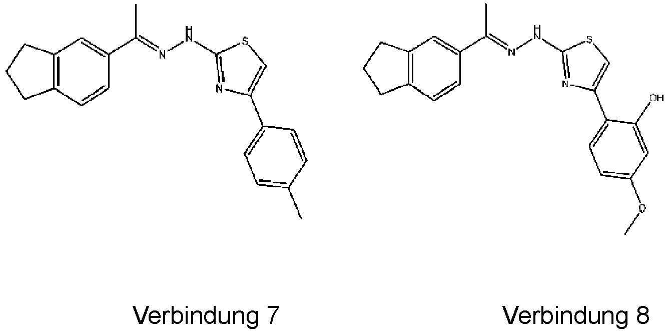

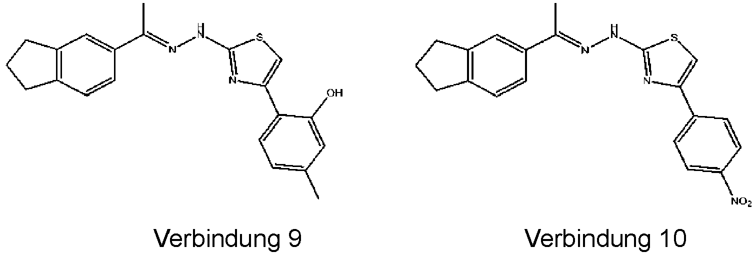

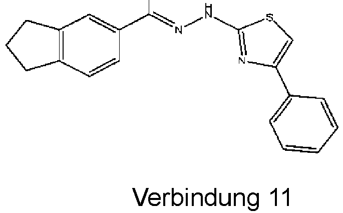

2. Verbindung zur Verwendung nach Anspruch 1 in der Antituberkulosebehandlung.

3. Verfahren zum Lösen von Verbindungen der unten angegebenen Struktur in Wasser, 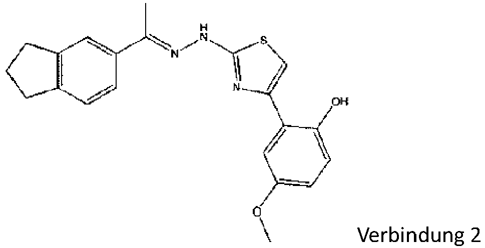 **dadurch gekennzeichnet, dass** es die folgenden Verfahrensschritte umfasst;
• Auflösen der Substanzen der Verbindung 2: Polysorbat 80 (Tween 80) im Massenverhältnis 1:6 in 7 ml Methanol,
• Verdampfen derselben bei 175 U/min, einer Temperatur von 40 °C und einem Vakuum von 140 ATM,
• Zugabe von Wasser zu dem erhaltenen Film und Mischen des Films mit einem Vortex,
• 15-minütiges Ruhen des Films in einem Ultraschallbad.

4. Eine Verbindung zur Verwendung nach Anspruch 1, wobei die maximale Dosis der Verbindung 1 zur Anti-Tuberkulose-Behandlung 1000 mg/kg beträgt.

## Revendications

1. Composé pour être utilisé dans le traitement de la tuberculose, dans lequel le composé est choisi dans le groupe constitué de:

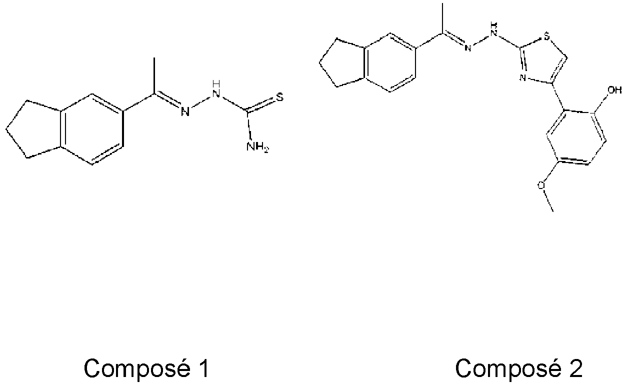

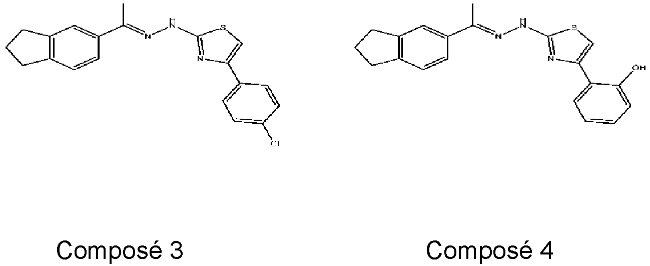

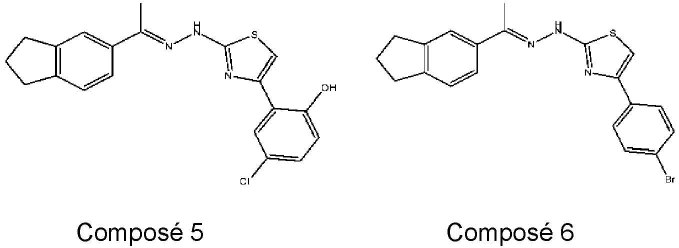

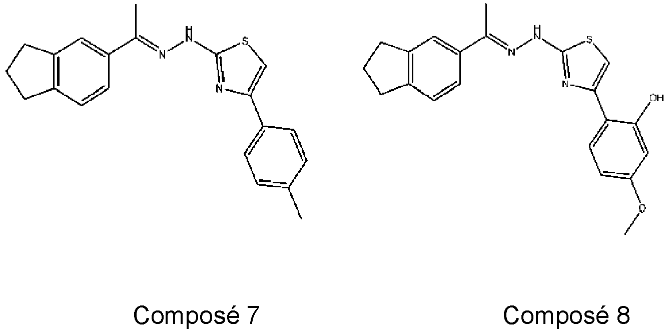

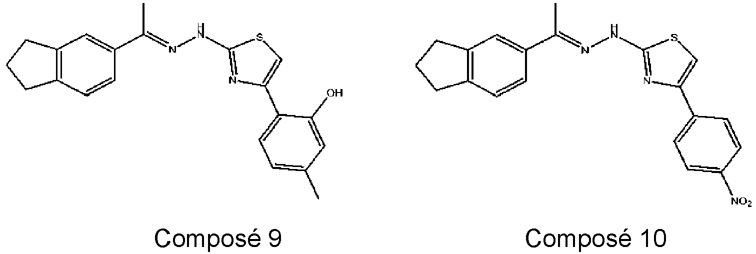

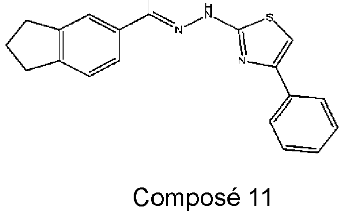

2. Composé pour être utilisé selon la revendication 1 dans le traitement antituberculeux.

3. Méthode pour dissoudre le composé de structure ci-dessous dans l'eau, 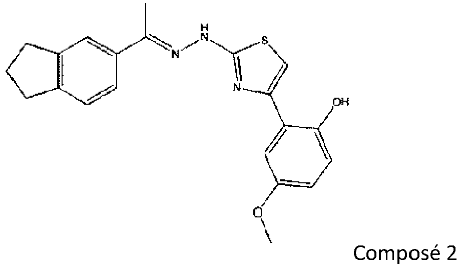 **Caractérisé en ce qu'**il comprend les étapes de processus suivantes;
• dissoudre les substances du Composé 2: Polysorbate 80 (Tween 80) au rapport massique de 1:6 dans 7 ml de méthanol,
• les évaporer à 175 RPM, à une température de 40°C et sous un vide de 140 ATM,
• ajouter de l'eau au film obtenu et mélanger le film avec vortex,
• laisser reposer le film 15 minutes dans un bain à ultrasons.

4. Composé pour être utiliser selon la revendication 1; dans lequel la dose maximale du Composé 1 pour le traitement antituberculeux est de 1000 mg/kg.
